# EUROPEAN PATENT APPLICATION

(11) **EP 1 908 481 A1**
(43) Date of publication of application: **09.04.2008**
(21) Application number: 06767219.6
(22) Date of filing: 23.06.2006
(51) Int. Cl.: A61K 45/00, A61K 31/5377, A61K 45/06, A61P 7/04, A61P 43/00

(54) **AGENT FOR REDUCTION OF BLEEDING IN CEREBROVASCULAR DISORDER**

(30) Priority: 24.06.2005 JP 2005185606
(71) Applicant: ONO PHARMACEUTICAL CO., LTD., Osaka-shi, Osaka 541-8526 (JP)
(72) Inventor: KITAJIMA, Takashi ONO PHARMACEUTICAL CO., LTD., Mishima-gun, Osaka 6188585 (JP); YASUHIRO, Tetsuya c/oONO PHARMACEUTICAL CO., LTD., Mishima-gun, Osaka 6188585 (JP); KAMOSHIMA, Wataru c/oONO PHARMACEUTICAL CO., LTD., Mishima-gun, Osaka 6188585 (JP); WAKAMATSU, Daisuke c/oONO PHARMACEUTICAL CO., LTD., Mishima-gun, Osaka 6188585 (JP)
(74) Representative: Henkel, Feiler & Hänzel
(86) International application number: PCT/JP2006/312569
(87) International publication number: WO 2006/137510

(57) **Abstract**

The present invention relates to a hemorrhage reducing agent in cerebrovascular disorder containing a poly (ADP- ribose) polymerase inhibitor (PARP inhibitor). The PARP inhibitor provides an inhibitory effect of vascular endothelial cell disorder so that it may reduce hemorrhage in cerebrovascular disorder. In addition, the PARP inhibitor inhibits the hemorrhage that is concerned about in thrombolytic agent use by using together with a thrombolytic agent, and an effect of extending therapeutic time window of a thrombolytic agent may be further expected. Furthermore, the PARP inhibitor can be a safe hemorrhage reducing agent with fewer side effects because it does not affect the blood coagulation system and the fibrinolytic system.

## Description

### FIELD OF THE INVENTION

The present invention relates to hemorrhage reducing agent in cerebrovascular disorder, containing a poly(ADP-ribose) polymerase (hereinafter abbreviated to PARP) inhibitor.

### BACKGROUND ART

The cerebrovascular diseases are classified roughly into two types, an ischemic cerebrovascular disease in which the blood flow to brain tissue decreases or ceases, and a hemorrhagic cerebrovascular disease in which the hemorrhage occurs in the skull. The ischemic cerebrovascular disease includes cerebral infarction, transient cerebral ischemic attack (TIA), and the like, and the hemorrhagic cerebrovascular disease includes brain hemorrhage, subarachnoid hemorrhage, and the like.

Cerebral infarction is nervous system disease, in which a cerebral blood vessel is occluded by arteriosclerosis of the cerebral blood vessel or with a thrombus carried by a blood flow from all but a brain, the blood flow is discontinued farther on the occluded part, and the nutrient supply for a brain cell is discontinued to ultimately cause cell death of a nerve cell. In addition, even if patient with cerebral infarction avoids sudden death, serious aftereffects by the functional disorder of nerve cells such as paralysis or aphasia are often left. In general, in a treatment of cerebral infarction, it is considered that the restarting of the bloodstream to flow needs to be performed soon after brain bloodstream was intercepted and before the brain tissue causes an irreversible change and fall into necrosis. A treatment method in the acute period of cerebral infarction is selected from antithrombotic therapy (thrombolytic therapy, anticoagulant therapy and antiplatelet therapy), cerebroprotection therapy, anticerebral edema therapy and hemodilution therapy according to the time after the onset of the symptom, disease severity and the clinical feature. Recently, a combination therapy of an antithrombotic agent and a cerebroprotection agent has been becoming the mainstream. Of these therapies, the therapy whose effect can be most expected is thrombolytic therapy. The thrombolytic therapy is a therapy in which a thrombolytic agent is administered in vivo, thereby dissolving morbid thrombus and reperfusing blood into ischemic tissue. For example, tissue plasminogen activator (t-PA) as a thrombolytic agent is effective for the patient within 3 hours after the onset of cerebral infarction. In this case, it is also reported that there are less aftereffects. However, if blood flow through the blood vessel disordered due to ischemia, it may be ruptured to bleed and blood coagulation disorder caused by a thrombolytic agent, result in promoting this hemorrhage. Thus, there is concern that the thrombolytic agent may induce hemorrhagic complication. Therefore, therapeutic time window (hereinafter abbreviated to TTW) of thrombolytic therapy is short as 3 to 6 hours. For example, in t-PA, a standard for use is provided, in which the thrombolytic agent should be used in the case where early ischemia-related change by the CT scan and intracranial hemorrhage are not recognized, it is within 3 hours after the onset of the symptom and the like.

Also as the therapy for cerebral infarction, in addition to the agent therapies, surgical treatments may be performed, such as percutaneous transluminal angioplasty by means of balloon catheter, stent placement and thrombectomy by means of catheter. However, the restarting of a rapid bloodstream to flow due to reperfusion after ischemia may cause hemorrhage from the blood vessel disordered due to the ischemia.

In contrast, the PARP inhibitor is reported to be useful as a cell death inhibitor because it has an effect of inhibiting failure of energy production system by abnormal activation of PARP. For example, a compound represented by formula (I): 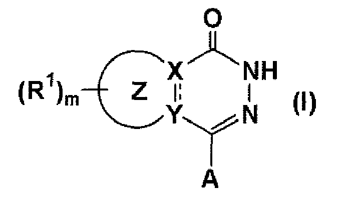 wherein all symbols are as defined hereinafter; has the PARP inhibitory activity. Therefore, it is useful as an agent for prevention and/or treatment for ischemic diseases (such as cerebral infarction, myocardial infarction, reperfusion damage and postoperative damage), inflammatory diseases (such as inflammatory bowel disease, multiple cerebral sclerosis, arthritis and lung disorder), neurodegenerative diseases (such as extrapyramidal system disorder, Parkinson's disease, Alzheimer's disease, muscular dystrophy and lumbar spinal stenosis), glaucoma, diabetes mellitus, complications of diabetes mellitus, shock, head injury, spinal cord injury, renal insufficiency, hyperalgesia and bloodstream disorder. In addition, it is disclosed that the compound is also useful as an antiretrovirus agents (HIV), a sensitizer of an anticancer therapy and an immunosuppressive agent (ref. Patent Document 1). However, there is neither description nor suggestion that the PARP inhibitor reduces hemorrhage in cerebrovascular disorder.
Patent Document 1: WO03/070707

### DISCLOSURE OF THE INVENTION

### Problem to be solved by the invention

It is required to develop a hemorrhage reducing agent in cerebrovascular disorder, which is safe and useful as a medicament.

### Means for solving the problem

The present inventors have intensively studied to solve the above problem and found for the first time that a PARP inhibitor reduces hemorrhage in cerebrovascular disorder. Also, the present inventors have found that a PARP inhibitor can serve as a safe and useful hemorrhage reducing agent without influencing blood coagulation system and fibrinolytic system because the PARP inhibitor has the effect of protecting vascular endothelial cell, and does not influence bleeding time. Thus, the present invention has been completed.

Namely, the present invention relates to the followings:
1. A hemorrhage reducing agent in cerebrovascular disorder, comprising a poly(ADP-ribose) polymerase inhibitor;
2. The agent according to the above 1, wherein the poly(ADP-ribose) polymerase inhibitor is one or more compound(s) selected from GPI-15427; GPI-16539; GPI-18078; GPI-6000; GPI-6150; KU-0687; INO-1001; FK-866; 4-(4-(N,N-dimethylaminomethyl)phenyl)-5-hydroxyisoquinolinone, FR-255595; FR-257516; FR-261529; FR-247304; M-50916; ABT-472; ONO-1924H; DR-2313; CEP-8983; AG-014699; BGP-15; AAI-028; PD-141076; PD-141703; a compound described in specifications of WO03/070707, WO00/044726, WO00/42,040, WO01/16136, WO01/42219 and WO01/70674;
3. The agent according to the above 1, wherein the poly(ADP-ribose) polymerase inhibitor is a compound represented by formula (I): 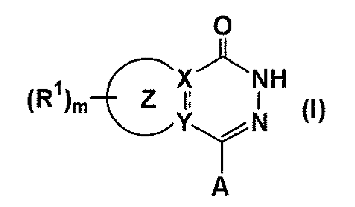 wherein R¹ represents (1) hydrogen atom, (2) C1-8 alkyl, (3) C1-8 alkoxy, (4) hydroxyl, (5) halogen atom, (6) nitro, (7) NR²R³, (8) C2-8 acyl, (9) C1-8 alkoxy substituted with phenyl, or (10) C2-8 acyl substituted with NR²R³; R² and R³ each independently represents (1) hydrogen atom or (2) C1-8 alkyl; X and Y are each independently represents (1) C, (2) CH or (3) N;
   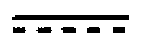 represents (1) a single bond or (2) a double bond; 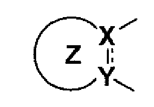 represents (1) C3-10 monocyclic carbocyclic aryl, which may be partially or fully saturated, or (2) 3- to 10-membered monocyclic heterocyclic aryl, which may be partially or fully saturated, containing 1 to 4 heteroatom(s) which is selected from oxygen atom, nitrogen atom and sulfur atom; and A represents (1) A¹, (2) A², (3) A³, (4) A⁴ or (5) A⁵;
   A¹ represents 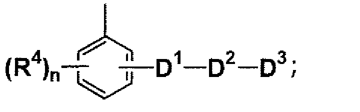 A² represents -E¹-E²-E³-E⁴;
   A³ represents 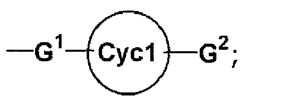 A⁴ represents 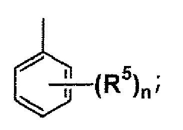 A⁵ represents 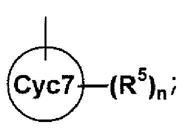 D¹ represents (1) -NR⁶C(O)-, (2) -NR⁶C(S)-, (3) -NR⁶SO₂-, (4) -CH₂-NR⁶-group, (5) -CH₂-O-, (6) -OC(O)-, (7) -CH₂-NR⁶C(O)-, (8) -NR⁶C(O)NR⁷-, (9) -NR⁶C(O)O-, (10) -NR⁶C(S)NR⁷-, (11) -NR⁶-, or (12) -NR⁶C(=NR⁷)-; R⁶ and R⁷ each independently represents (1) hydrogen atom, (2) C1-8 alkyl, (3) phenyl or (4) C1-8 alkyl substituted with phenyl; D² represents (1) C1-8 alkylene, (2) C2-8 alkenylene, (3) Cyc2, (4) -(C1-4 alkylene)-O-(C1-4 alkylene)-, (5) -(C1-4 aikylene)-S-(C1-4 alkylene)-, (6) -(C1-4 alkylene)-NR⁸-(C1-4 alkylene)-, (7) -(Cyc2)-(C1-8 alkylene)-, (8) -(C1-8 alkylene)- (Cyc2)-, or (9) -(C1-4 alkylene)-(Cyc2)-(C1-4 alkylene)-; R⁸ represents (1) hydrogen atom, (2) C1-8alkyl, (3) C1-8 alkoxycarbonyl, (4) phenyl or (5) C1-8 alkyl substituted with phenyl; D³ represents (1) hydrogen atom, (2) -NR⁹R¹⁰, (3) Cyc3, (4) -OR¹¹, (5) COOR¹², (6) CONR¹³R¹⁴, (7) cyano, (8) halogen atom, (9) -C(= CR¹⁵)NR¹⁶R¹⁷, or (10) -NR¹⁸C(= NR¹⁹)NR²⁰R²¹; R⁹ and R¹³ each independently represents (1) hydrogen atom, (2) C1-8 alkyl, (3) C2-8 alkenyl, (4) C2-8 alkynyl, (5) Cyc3, (6) C1-8 alkoxy, (7) C2-8 alkenyloxy, (8) C2-8 alkinyloxy, or (9) C1-8 alkyl substituted with Cyc3, C1-8 alkoxy, C1-8 alkylthio, cyano, hydroxyl or 1 to 3 halogen atom(s); R¹⁰ and R¹⁴ each independently represents (1) hydrogen atom, (2) Cl-8 alkyl, (3) C2-8 alkenyl, (4) C2-8 alkynyl, (5) C1-8 alkoxycarbonyl, (6) C2-8 acyl, (7) C3-8 cycloalkyl, (8) C1-8 alkoxycarbonyl substituted with Cyc4 or 1 to 3 halogen atom(s), or (9) C1-8 alkyl substituted with C1-8 alkoxy; R¹¹ and R¹² each independently represents (1) hydrogen atom or (2) C1-8 alkyl; R¹⁵, R¹⁶, R¹⁷, R¹⁸, R¹⁹, R²⁰ and R²¹ each independently represents (1) hydrogen atom, (2) C1-8 alkyl, (3) C1-8 alkoxycarbonyl, (4) phenyl or (5) C1-8 alkyl substituted with phenyl; R⁴ represents (1) hydrogen atom, (2) C1-8 alkyl, (3) C1-8 alkoxy, (4) hydroxyl, (5) halogen atom, (6) nitro or (7) NR²²R23; R²² and R²³ each independently represents (1) hydrogen atom or (2) C1-8 alkyl; E¹ represents C1-4 alkylene, E² represents (1) -C(O) NR²⁴-, (2) -NR²⁴C (O)-, (3) -NR²⁴-, (4) -C(O)O- or (5) -S-; R²⁴ represents (1) hydrogen atom, (2) C1-8 alkyl or (3) C1-8 alkyl substituted with phenyl; E³ represents (1) a single bond or (2) C1-8 alkylene; E⁴ represents (1) C1-8 alkyl, (2) C2-8 alkenyl, (3) C2-8 alkynyl, (4) Cyc5, (5) NR²⁵R²⁶, (6) OR²⁷, (7) SR²⁷, (8) COOR²⁷, (9) C1-8 alkyl substituted with two OR²⁵S, (10) C1-8 alkyl substituted with 1-3 halogen atoms, (11) cyano, or (12) C2-8 acyl; R²⁵ represents (1) hydrogen atom, (2) C1-8 alkyl, (3) C2-8 alkenyl, (4) C2-8 alkynyl, (5) Cyc5 or (6) C1-8 alkyl substituted with Cyc5 or OR²⁸; R²⁶ represents (1) hydrogen atom, (2) C1-8 alkyl, (3) C1-8 alkoxycarbonyl, (4) phenyl or (5) C1-8 alkyl substituted with phenyl; R²⁷ represents (1) hydrogen atom, (2) C1-8 alkyl, (3) Cyc5 or (4) C1-8 alkyl substituted with Cyc5; R²⁸ represents (1) hydrogen atom or (2) C1-8 alkyl; G¹ represents C1-8 alkylenes; Cyc1 represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom; G² represents (1) hydrogen atom, (2) C1-8 alkyl, (3) C1-8 alkoxycarbonyl, (4) C2-8 acyl, (5) Cyc6, (6) C1-8 alkyl or C2-8 alkenyl substituted with 1 to 2 of Cyc6, hydroxyl or the C1-8 alkoxy, (7) C1-8 alkoxycarbonyl substituted with Cyc6, (8) -C(O)-Cyc6, (9) nitro, (10) NR⁴¹R⁴², (11) C1-8 alkoxy, or (12) C1-8 alkyl substituted with NR⁴¹R⁴²; R⁴¹ and R⁴² each independently represents (1) hydrogen atom or (2) C1-8 alkyl; R⁵ (1) hydrogen atom, (2) C1-8 alkyl, (3) C1-8 alkoxy, (4) hydroxyl, (5) nitro, (6) NR²⁹R³⁰, (7) C1-8 alkyl substituted with NR²⁹R³⁰, (8) NHSO₂OH, (9) amidino, (10) cyano, (11) halogen atom, (12) Cyc8, or (13) C1-8 alkyl substituted with Cyc8; R²⁹ and R³⁰ each independently represents (1) hydrogen atom or (2) C1-8 alkyl; Cyc2, Cyc3, Cyc4, Cyc5, Cyc6 and Cyc8 each independently represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom; Cyc7 represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom, provided that Cyc7 does not represent benzene ring, Cyc2, Cyc3, Cyc4, Cyc5, Cyc6 and Cyc8 may be substituted with 1 to 3 group(s), which is selected from (1) C1-8 alkyl, (2) C2-8 alkenyl, (3) C1-8 alkoxy, (4) halogen atom, (5) trihalomethyl, (6) trihalomethoxy, (7) C1-8 alkoxycarbonyl, (8) oxo, (9) C1-8 alkyl substituted with the C1-8 alkoxy or phenyl, (10) hydroxyl, or (11) NR²⁹R³⁰; and m and n each independently represents 1 or 2, or a salt, an N-oxide or solvate thereof, or a prodrug thereof;
4. The agent according to the above 3, wherein the compound represented by formula (I) is 4-(N-(4-(morpholin-4-yl)butyl)carbamoylmethyl)-5,6,7,8-tetrahydrophthalazin-1(2H)-one;
5. The agent according to the above 1, wherein the hemorrhage is caused by antithrombotic treatment;
6. The agent according to the above 1, wherein the hemorrhage is caused by one or more drug(s) selected from a thrombolytic agent, an anticoagulant agent and an antiplatelet agent;
7. The agent according to the above 1, which is an agent for inhibiting vascular endothelial cell disorder;
8. The agent according to the above 7, which is an agent for repair and/or disrupting inhibition of blood-brain barrier;
9. The agent according to the above 1, which is an agent for extending therapeutic time window of a thrombolytic agent;
10. The agent according to the above 1, which is an agent for prevention and/or treatment of cerebrovascular disorder;
11. The agent according to the above 1, which is an agent for prevention and/or treatment of hemorrhagic complication due to antithrombotic treatment;
12. A hemorrhage reducing agent in cerebrovascular disorder, comprising a poly(ADP-ribose) polymerase inhibitor used in combination with one or more agent(s) selected from a thrombolytic agent, an anticoagulant agent, an antiplatelet agent, a cerebroprotection agent, an anticerebral edema agent, a plasma expander, an immunosuppressive agent, an intercellular adhesion factor inhibitor, an interleukin-8 antagonist and a steroid;
13. The agent according to the above 12, wherein the agent is a concomitant agent;
14. The agent according to the above 13, wherein the poly(ADP-ribose) polymerase inhibitor is used in combination with one or more agent(s) selected from a thrombolytic agent, an anticoagulant agent and an antiplatelet agent;
15. The agent according to the above 14, wherein the poly(ADP-ribose) polymerase inhibitor is 4-(N-(4-(morpholin-4-yl)butyl)carbamoylmethyl)-5,6,7,8-tetrahydrophthalazin-1(2H)-one, and the thrombolytic agent is t-PAs;
16. The agent according to the above 1, wherein the hemorrhage is bleeding in a hemorrhagic cerebrovascular disease and/or bleeding involved in ischemic-reperfusion;
17. The agent according to the above 1, which is an agent for protecting a vascular endothelial cell;
18. A method for reducing hemorrhage in cerebrovascular disorder in mammals, comprising administering effective dose of a poly(ADP-ribose) polymerase inhibitor to the mammals;
19. A use of a poly(ADP-ribose) polymerase inhibitor for producing a hemorrhage reducing agent in cerebrovascular disorder;
20. A method for reducing hemorrhage in cerebrovascular disorder in mammals, comprising administering effective dose of 4-(N-(4-(morpholin-4-yl)butyl)carbamoylmethyl)-5,6,7,8-tetrahydrophthalazin-1(2H)-one used in combination with effective dose of t-PAs to mammals; and
21. A use of 4-(N-(4-(morpholin-4-yl)butyl)carbamoylmethyl)-5,6,7,8-tetrahydrophthalazin-1(2H)-one and t-PAs for producing a hemorrhage reducing agent in cerebrovascular in which one or more agent(s) are used in combination.

### BEST MODE FOR CARRYING OUT THE INVENTION

A PARP inhibitor may be any compound which inhibits the PARP activity, and includes not only known PARP inhibitors, but also all PARP inhibitors which will be found in future.

The PARP inhibitor includes such as the compound represented by formula (I), GPI-15427, GPI-16539, GPI-18078, GPI-6000, GPI-6150, KU-0687, INO-1001, FK-866, 4-(4-(N,N-dimethylaminomethyl)phenyl)-5-hydroxyisoquinolinone, FR-255595, FR-257516, FR-261529, FR-247304, M-50916, ABT-472, ONO-1924H, DR-2313, CEP-8983, AG-014699, BGP-15, AAI-028, PD-141076, PD-141703, the compounds described in specifications of WO03/070707, WO00/044726, WO00/42040, WO01/16136, WO01/42219, WO01/70674, etc.

The compound represented by formula (I) preferably includes 4-(N-(4-(morpholin-4-yl)butyl)carbamoylmethyl)-5,6,7,8-tetrahydrophthalazin-1(2H)-one, or a salt, an N-oxide, or solvate thereof, or a prodrug thereof.

In this specification, the cerebrovascular disorder collectively represents cerebral disorders and/or nervous system disorders which are induced by an abnormality of cerebral blood vessel, including ischemic cerebrovascular disease (cerebral infarction (such as cerebral thrombosis,and cerebral embolism), transient cerebral ischemic attack (such as TIA)), hemorrhagic cerebrovascular disease (such as cerebral hemorrhage and subarachnoid hemorrhage), moyamoya disease, vascular malformation (such as cavernous hemangioma and venous angioma), idiopathic carotid-cavernous fistula, chronic subdural hematoma, etc.

The hemorrhage in cerebrovascular disorder may be seen in brain, stomach, intestine, kidney, lung and the like. The hemorrhage reducing agent in the present invention is in particular the one for hemorrhage in the brain.

The hemorrhage in cerebrovascular disorder includes all hemorrhages in the brain such as bleeding in the hemorrhagic cerebrovascular disease, bleeding involved in ischemia-reperFusion, etc. Further, the reperfusion includes natural reperfusion, and reperfusion due to antithrombotic treatment. The antithrombotic treatment includes thrombolytic therapy, anticoagulation therapy, antiplatelet therapy, and surgical treatment such as percutaneous transluminal angioplasty by means of balloon catheter, stent placement, and thrombectomy by means of catheter, etc.

In the thrombolytic therapy, anticoagulation therapy and antiplatelet therapy, a thrombolytic agent, an anticoagulation agent and an antiplatelet agent are used, respectively.

The thrombolytic agent includes such as t-PAs (t-PA, alteplase, tisokinase, nateplase, pamiteplase, monteplase, desmoteplase, etc), urokinase, prourokinase, nasaruplase, streptokinase, etc.

The anticoagulant agent includes such as Heparins (heparin sodium, heparin calcium, Heparinoid, low-molecular-weight heparins (parnaparin, dalteparin, danaparoid, enoxaparin, nadroparin, bemiparin, reviparin, tinzaparin, etc.), etc.), activated Factor X inhibitors (fondaparinux), DX-9065a, DU-176b, CS-3030, JTV-803, BMS-561389, BAY-59-7939, YM150, LY-517717, KFA-1982, KFA-1829, idraparinux, DPC-423, DPC-602, DPC-A52350, Otamixaban, HMR2096, FXV-673, RPR-130673, MCM16, MCM17, TC-10, RPR-256580, RPR-225430, RPR-247978, RPR-231352, RPR-209685, RPR-208944, RPR-208815, RPR-208707, RPR-208566, RPR-200095, RPR-130338, RPR-130737, RPR-132747, RPR-128515, RPR-120844, M-55113, M-55190, M-55555, M-55529, MLN-1021, EGR-Xa, CI-1031, ZD-5227, AX-1826, ZK-813039, DE-00684, BIBT-986, BIBT-1011, BM-141248, PD-198961, PD-0313052, PD-313052, PMD-3112, PMD-3833, PMD-3805, PMD-3829, PMD-2612, PMD-2837, PMD-2566, SEL-2711, SSR-122497A, SSR-126517, SSR-128428, SSR-128429, SSR-80670A, SSR-121903A, SSR-122429A, SSR-122574A, Org-42675, etc.), activated factor IX inhibitor (TTP-889, 224AE3, etc.), vitamin K antagonists (warfarin, etc.), antithrombin agents (argatroban, gabexate mesilate, nafamostat mesilate, ximelagatran, melagatran, dabigatran, bivalirudin, lepirudin, hirudin, desirudin, SSR-182289A, SR-123781A, S-18326, AZD-0837, LB-30870, L-375378, MCC-977, AT-1362, etc.), activated protein C preparations (human activated protein C), antithrombin III preparations, tissue factor pathway inhibitors, thrombomodulin preparations (ART-123, MR-33, etc.), carboxypeptidase U inhibitors, thrombin-activatable fibrinolysis inhibitors (TAFI) (sodium citrate, AZD-9684, etc.), etc.

The anti platelet agent includes such as aspirin, ticlopidine, clopidogrel, dipyridamole, cilostazol, ozagrel, prasugrel, ethyl icosapentate, beraprost, sarpogrelate, limaprost, GPIIb/IIIa receptor antagonists (abciximab, tirofiban, eptifibatide, YM028, etc.), AZD6140, etc.

In the present invention, the protection of vascular endothelial cell means the maintenance of the normal function in the vascular endothelium cell. For example, the protection of vascular endothelial cell includes the prevention of vascular endothelial cell from being disordered, being damaged, or getting denatured and further causing cell death due to some reasons. In addition, in the case of the physically normal vascular endothelial cell, it is included as a target for the vascular endothelial cell protecting agent of the present invention, as far as it is the vascular endothelial cell becoming functionally abnormal. The vascular endothelial cell protecting effect includes the inhibitory effect of vascular endothelial cell disorder.

In this specification, the vascular endothelial cell disorder includes death of vascular endothelial cell or functional disorder of vascular endothelial cell. The function of vascular endothelial cell includes, but not limited to, for example, the maintenance of blood vessel flexibility, prevention of blood cell leak, vasoconstriction and vasodilation, or prevention of thrombosis. Further, the vascular endothelial cell disorder also includes a blood-brain barrier disorder. A PARP inhibitor has an inhibitory effect of the vascular endothelial cell disorder so that repair effect on the blood-brain barrier disorder and inhibitory effect on the blood-brain barrier disruption can be expected.

In this specification, therapeutic time window (TTW) means the time frame, in which treatment can be performed without leaving most of the aftereffects. It is considered that TTW of a thrombolytic agent is about 3 to 6 hours, for example, as for t-PA, its use is authorized for a case within 3 hours after the onset of the symptom of cerebral infarction in the United States.

In this specification, the C1-8 alkyl includes, for example, methyl, ethyl, propyl, butyl, pentyl, hexyl, heptyl, octyl, and isomer groups thereof.

In this specification, the C2-8 alkenyl includes, for example, ethenyl, propenyl, butenyl, pentenyl, hexenyl, heptenyl, octenyl, and isomer groups thereof.

In this specification, the C2-8 alkynyl includes, for example, ethynyl, propynyl, butynyl, pentynyl, hexynyl, heptynyl, octynyl, and isomer groups thereof.

In this specification, the C1-8 alkoxy includes, for example, methoxy, ethoxy, propoxy, butoxy, pentyloxy, hexyloxy, heptyloxy, octyloxy, and isomer groups thereof.

In this specification, the C2-8 alkenyloxy includes, for example, ethenyloxy, propenyloxy, butenyloxy, pentenyloxy, hexenyloxy, heptenyloxy, octenyloxy, and isomer groups thereof.

In this specification, the C2-8 alkynyloxy includes, for example, ethynyloxy, propynyloxy, butynyloxy, pentynyloxy, hexynyloxy, heptynyloxy, octynyloxy, and isomer groups thereof.

In this specification, the C1-8 alkylthio includes, for example, methylthio, ethylthio, propylthio, butylthio, pentylthio, hexylthio, heptylthio, octylthio, and isomer groups thereof.

In this specification, the C1-4 alkylene includes, for example, methylene, ethylene, trimethylene, tetramethylene, and isomer groups thereof.

In this specification, the C1-8 alkylene includes, for example, methylene, ethylene, trimethylene, tetramethylene, pentamethylene, hexamethylene, heptamethylene, octamethylene and isomer groups thereof.

In this specification, the C2-8 alkenylene includes, for example, ethenylene, propenylene, butenylene, pentenylene, hexenylene, heptenylene, octenylene, and isomer groups thereof.

In this specification, the C1-8 alkoxycarbonyl includes, for example, methoxycarbonyl, ethoxycarbonyl, propoxycarbonyl, butoxycarbonyl, pentyloxycarbonyl, hexyloxycarbonyl, heptyloxycarbonyl, octyloxycarbonyl, and isomer groups thereof.

In this specification, trihalomethyl includes, for example, methyl substituted by three halogen atoms.

In this specification, trihalomethoxy includes, for example, methoxy substituted by three halogen atoms.

In this specification, the C2-8 acyl includes, for example, ethanoyl (acetyl), propanoyl (propionyl), butanoyl (butyryl), pentanoyl (valeryl), hexanoyl, heptanoyl, octanoyl, and isomer groups thereof.

In this specification, the C3-8 cycloalkyl includes, for example, cyclopropyl, cyclobutyl, cyclopentyl, cyclohexyl, cycloheptyl, cyclooctyl.

In this specification, halogen atom includes chlorine, bromine, fluorine, and iodine.

In this specification, the C3-10 monocyclic carbocyclic aryl, which may be partially or fully saturated, represented by 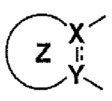 includes, for example, cyclopropane, cyclobutane, cyclopentane, cyclohexane, cycloheptane, cyclooctane, cyclononane, cyclodecane, cyclopropene, cyclobutene, cyclopentene, cyclohexene, cycloheptene, cyclooctene, cyclonone, cyclodecene, cyclohexadiene, cycloheptadiene, cyclooctadiene, etc.

In this specification, the 3- to 10-membered monocyclic heterocyclic aryl, which may be partially or fully saturated, containing 1 to 4 heteroatom(s) which is selected from oxygen atom, nitrogen atom and sulfur atom represented by 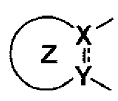 includes aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiine (dihydrothiopyran), tetrahydrothiine (tetrahydrothiopyran), dihydrothiepine, tetrahydrothiepine, perhydrothiepine, dihydrooxazole, tetrahydrooxazole (oxazalidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, dithiane, etc.

In this specification, the 3- to 10-membered monocyclic or bicyclic heterocyclic aryl containing 1 to 4 heteroatom(s) which is selected from oxygen atom, nitrogen atom and sulfur atom among the 3- to 10-membered monocyclic or bicyclic heterocyclic aryl, which may be partially or fully saturated, containing 1 to 4 heteroatom(s) which is selected from oxygen atom, nitrogen atom and sulfur atom represented by Cyc1, Cyc2, Cyc3, Cyc4, Cyc5, Cyc6, Cyc7, and Cyc8 includes pyrrole, imidazole, triazole, tetrazole, pyrazole, pyridine, pyrazine, pyrimidine, pyridazine, azepine, diazepine, furan, pyran, oxepine, thiophene, thiine, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, etc.

Furthermore, the 3- to 10-membered monocyclic or bicyclic heterocyclic aryl, which is partially or fully saturated, containing 1 to 4 heteroatom(s) which is selected from oxygen atom, nitrogen atom and sulfur atom includes aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiine (dihydrothiopyran), tetrahydrothiine (tetrahydrothiopyran), dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazalidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dithiolane, dithiane, dioxaindan, benzodioxane, chroman, benzodithiolane, benzodithiane, etc.

The above described heterocyclic rings include N-oxide thereof where nitrogen of the above described heterocyclic rings is oxidized nitrogen atom.

In this specification, the 3- to 10-membered monocyclic heterocyclic aryl, which may be partially or fully saturated, containing 1 to 4 heteroatom(s) which is selected from oxygen atom, nitrogen atom and sulfur atom represented by Cyc1, Cyc2, Cyc3, Cyc4, Cyc5, Cyc6, Cyc7, and Cyc8 includes cyclopropane, cyclobutan, cyclopentane, cyclohexane, cycloheptane, cyclooctane, cyclononane, cyclodecane, cyclopropene, cyclobutene, cyclopentene, cyclohexene, cycloheptene, cyclooctene, cyclononene, cyclodecene, cyclopentadiene, cyclohexadiene, cycloheptadiene, cyclooctadiene, benzene, pentalene, azulene, perhydroazulene, perhydropentalene, indene, perhydroindene, indan, naphthalene, teterahydronaphthalene, perhydronaphthalene, etc., provided that Cyc7 does not represent benzene.

According to the present invention, symbol 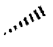 represents a bond to the opposite side of the paper (i.e., α-configuration), symbol 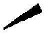 represents a bond to front side of the paper (i.e., β-configuration), and symbol 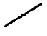 represents a mixture of α- and β-configurations.

Unless otherwise specifically mentioned, all isomers are included in the present invention. For example, alkyl, alkenyl, alkynyl, alkoxy, alkylthio, alkylene, alkenylene and alkynylene include straight chain and branched one. Moreover, all of isomers due to double bond, ring and fused ring (E-, Z-, cis- and trans-forms), isomers due to presence of asymmetric carbon(s) etc. (R-, S-, α- and β-configuration, enantiomer and diastereomer), optically active substances having optical rotation (D-, L-, d- and I-forms), polar compound by chromatographic separation (more polar compound and less polar compound), equilibrium compounds, rotational isomers, a mixture thereof in any proportion and a racemic mixture are included in the present invention.

Among salts, a water-soluble salt is preferred. Examples of appropriate salts are salt with alkaline metal (potassium, sodium, lithium, etc.), salt with alkaline earth metal (calcium, magnesium, etc.), ammonium salt, pharmaceutically acceptable salt with organic amine (tetramethylammonium, triethylamine, methylamine, dimethylamine, cyclopentylamine, benzylamine, phenethylamine, piperidine, monoethanolamine, diethanolamine, tris(hydroxymethyl) methylamine, lysine, arginine, N-methyl-D-glucamine, etc.), etc.

Among acid addition salts, a water-soluble salt is preferred. Appropriate acid addition salts include such as inorganic acid salt (hydrochloride, hydrobromide, hydroiodide, sulfate, phosphate and nitrate, etc.), organic acid salt (acetate, trifluoroacetate, lactate, tartrate, oxalate, fumarate, maleate, benzoate, citrate, methanesulfonate, ethanesulfonate, benzenesulfonate, toluenesulfonate, isothionate, glucuronate and gluconate, etc.), etc.

The compounds represented by formula (I) and salts thereof may be converted to solvates by known methods. The solvate is preferably low-toxic and water-soluble. Appropriate solvates include such as solvates with water and with alcoholic solvent (ethanol, etc.).

Moreover, the salt includes a quaternary ammonium salt. The quaternary ammonium salt of the compound represented by formula (I) is the compound where nitrogen of the compounds represented by formula (I) is quarternalized by R° (R° is C1-8 alkyl or C1-8 alkyl substituted by phenyl.).

The compound of the present invention can be converted into an N-oxide by known methods. The N-oxide is the compound where nitrogen of the compound represented by formula (I) is oxidized.

A prodrug of the compound of formula (I) means a compound which is converted to the compound of formula (I) by reaction with an enzyme, gastric acid or the like in the living body. For example, with regard to a prodrug of the compound of formula (I), when the compound of formula (1) has an amino group, compounds in which the amino group is, for example, acylated, alkylated or phosphorylated (e.g., compounds in which the amino group of the compound of formula (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, tert-butylated, etc.); when the compound of formula (I) has a hydroxyl group, compounds where the hydroxyl group is, for example, acylated, alkylated, phosphorylated or borated (e.g., compounds in which the hydroxyl group of the compound of formula (I) is acetylated, palmitoylated, propanoylated, pivaloylated, succinylated, fumarylated, alanylated or dimethylaminomethylcarbonylated); and when the compound of formula (I) has a carboxyl group, the compound in which the carboxyl group is, for example, esterified or amidated (e.g., compounds in which the carboxyl group of the compound of formula (I) is made into ethyl ester, phenyl ester, phenylethyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide). Those compounds may be produced by a known method per se. The prodrug of the compound of formula (I) may be either a hydrate or a non-hydrate. A prodrug of the compound of formula (I) may also be a compound which is converted to the compound of formula (I) under physiologic condition as described in "Iyakuhin no kaihatsu, Vol.7 (Bunshi-sekkei), pp.163-198 (Hirokawa-Shoten), 1990". And the compound of formula (I) may also be labeled by a radio isotope (such as ³H, ¹⁴C, ³⁵S, ¹²⁵I, etc.).

### Method for Producing PARP Inhibitor

A PARP inhibitor used for the present invention may be produced by using known methods, methods described in specifications of WO03/070707, WO00/044726, WO00/42040, WO01/16136, WO01/42219 and WO01/70674, or methods obtained by appropriately modifying similar methods used in combination. Also, the compound represented by formula (I) can be produced by using the method described in specification of WO03/070707, or methods obtained by appropriately modifying similar methods used in combination.

### Toxicity

A PARP inhibitor used in the present invention, for example, a compound represented by formula (I) has very low toxicity and is sufficiently safe suited for as a medicament.

### Application to Pharmaceuticals

A PARP inhibitor can reduce hemorrhage in cerebrovascular disorder, because it has the effect on the vascular endothelial cell disorder. Thereby, the PARP inhibitor is useful for the prevention and/or treatment of cerebrovascular disorder caused secondarily due to intracerebral hemorrhage and/or hemorrhagic complications caused by antithrombotic treatment (antithrombotic therapy (thrombolytic therapy, anticoagulation therapy, antiplatelet therapy)) or surgical therapy (percutaneous transluminal angioplasty by means of balloon catheter, stent placement, thrombectomy by means of catheter, etc.).

The PARP inhibitor, in a case of using in combination with a thrombolytic agent, an anticoagulant agent and/or an antiplatelet agent, inhibits hemorrhage caused by these agents. Therefore, the extension of TTW of a thrombolytic agent can be expected (a thrombolytic agent has narrow TTW). Particularly, t-PAs are more effective in the combination with a PARP inhibitor, because t-PAs have short 3-hours therapeutic time window. Further, the PARP inhibitor has also the cerebral infarction inhibitory effect, therefore the enhancement of the cerebral infarction inhibitory effect can be expected, by means of a combination with a thrombolytic agent, an anticoagulation agent and/or an antiplatelet agent.

The PARP inhibitors such as compounds represented by formula (I) may be administered as a concomitant agent in combination with the other agent(s) for 1) complementation and/or potentiation of therapeutic effect of the compound; 2) improvement of kinetics and absorption of the compound, and reduction of dosage of the compound; and/or 3) reduction of side effects of the compound.

A concomitant agent of a PARP inhibitor and other agent(s) may be administered in a form of the combination agent that combined both ingredients in one formulation, or may adopt a form to administer as separate agents. The administration of these agents in a form of separate agents includes simultaneous administration and administration with different time intervals. In addition, in the administration with different time intervals, the PARP inhibitor may be administered in advance and other agent(s) may be administered later, or other agent(s) may be administered in advance and the PARP inhibitor may be administered later. Further, the respective administration method may be the same or different.

The other agent(s) may be a low molecular compound and may be macromolecular protein, polypeptide, polynucleotide (DNA, RNA, and gene), antisense, decoy, and antibody, vaccine or the like. A dosage of other agent(s) can be selected accordingly based on a dosage clinically used. In addition, the combination ratio of a PARP inhibitor and other agent(s) can be selected accordingly to the age and weight of a subject to be treated, the administration method, the administration time and the like. For example, the other agent(s) may be used in an amount of 0.01 to 100 parts by mass based on 1 part by mass of PARP inhibitor. The other agent(s) may be administered in combination with any one or more agent(s) selected from a similar group and a different group shown in the following, in an appropriate ratio.

The other agent(s) include, besides thrombolytic agents, anticoagulant agents or antiplatelet agents described above, cerebroprotection agents (radical scavengers (edaravone and ebselen (DR-3305)), astrocyte modulators (ONO-2506), N-methyl-D-aspartate (NMDA) antagonists, a-Amino-3-hydroxy-5-methylisooxazole-4-propionate (AMPA) antagonists, nitric oxide synthetase (NOS) inhibitors (L-NMMA, ONO-1714), calcium antagonists, sodium channel antagonists, opioid antagonists, GABA agonists and neurotrophic factor), anticerebral edema agents (glycerol and mannitol), plasma expanders (dextran 40, hydroxyethylated starch, albumin and pentastarch), immunosuppressive agents (cyclosporine, tacrolimus, azathioprine, methotrexate and cyclophosphamide), intercellular adhesion factor inhibitors, interleukin-8 antagonists, steroids and the like.

In addition, the other agent(s) which is for complementation and/or potentiation of therapeutic effect of the prevention and/or treatment of the PARP inhibitor such as the compound represented by formula (I), includes not only the agents which have already been found based on the mechanism described above, but also those which will be found in future.

The PARP inhibitor such as the compound represented by formula (I) may be formulated by optionally adding a pharmaceutically acceptable additive and using technologies used widely for preparation of a single agent or a combination agent.

In order to use the PARP inhibitor such as the compound represented by formula (I) used in the present invention or use a concomitant agent of the compounds represented by formula (I) with other agent(s) for the purpose described above, they are usually administered systematically or locally in an oral form or a parenteral form.

The doses to be administered are determined depending upon, for example, age, body weight, symptom, the desired therapeutic effect, the route of administration, the duration of the treatment, and the like. For a human adult, generally 1 mg to 1000 mg per dose is orally administered once to several times a day, or 1 mg to 100 mg per dose is parenterally (preferably intravenously) administered once to several times a day, or intravenously administered continuously for 1 to 24 hour(s) a day. Furthermore, more than two routes of administration may be used in combination.

In addition, for example, t-PA which has been already used clinically is administered parenterally, preferably intravenously at dose of 0.6 mg/kg or 0.9 mg/kg.

As mentioned above, the doses to be administered depend upon various conditions. Therefore, there may be cases where doses lower than or greater than the ranges specified above are applied.

When the PARP inhibitor such as the compound represented by formula (I) used in the present invention or the concomitant agent of the compound represented by formula (I) with other agent(s) is administered, they are used, for example, in the form of solid for oral administration, liquid form for oral administration and an injection for parenteral administration.

Solid forms for oral administration include compressed tablets, pills, capsules, dispersible powders, granules and the like. Capsules include hard capsules and soft capsules.

In such solid forms, one or more of the active compound(s) may be admixed with vehicles (such as lactose, mannitol, glucose, microcrystalline cellulose, starch or the like), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more layer. And further, coating may include containment within capsules of absorbable materials such as gelatin.

Liquid forms for oral administration include pharmaceutically acceptable solutions, suspensions, emulsions, syrups elixirs and the like. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

The injection for parenteral administration includes all forms of injections and also drops. For example, it includes intramuscular injection, subcutaneous injection, intracutaneous injection, an intraarterial injection, an intravenous injection, an intraperitoneal injection, intraspinal injection, intravenous drops, etc.

Injections for parenteral administration include, for example, sterile aqueous, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, saline, vegetable oil, propylene glycol, polyethylene glycol, alcohol such as ethanol, or a mixture thereof. Injections may further comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared according to sterile methods. They may also be manufactured in the form of sterile solid forms such as freeze-dried products, which may be dissolved in sterile water or some other sterile diluent(s) for injection immediately before use.

The PARP inhibitor such as the compound represented by formula (I) has the inhibitory effect of the vascular endothelial cell disorder, therefore can reduce hemorrhage in the cerebrovascular disorder. In addition, it is further expected that use of the PARP inhibitor in combination with other agent(s) such as thrombolytic agent can reduce hemorrhage by these agents and extend TTW of thrombolytic agent.

### EXAMPLES

### Biological Examples

### 1. Influence of PARP Inhibitor on Bleeding Time

A rat (male Sprague Dawley (SD) rat, 7 weeks old; Shimizu Laboratory Supplies Co., Ltd. (SLC))) was anesthetized with urethane (1.2 g/ 6 mL/kg, i.p.), and then the animal was placed on an incubation mat (Microtemp, Seabrook Medical Systems) set to 37°C after indwelling a catheter (JMS cutdown tube, JMS Co., Ltd.) made of vinyl chloride in a left femoral vein for administration of a test substance. Saline, 4-(N-(4-(morpholin-4-yl)butyl) carbamoylmethyl)-5,6,7,8-tetrahydrophthalazin-1(2H)-one (compound A) (30 mg/kg), or heparin (100 U/kg) which was positive control substance, was administered with an intravenous bolus. Five minutes after the administration, a tip of the tail was cut at the position of 2 mm with a razor (single-edged feather), and immediately, the tail was immersed in saline (Otsuka normal saline) warmed to 37°C. Finally, the time until the hemorrhage was stopped (30 minutes at a maximum) was measured, which was regarded as bleeding time.

As for the heparin treated group, hemorrhage lasted during a measurement time of 30 minutes whereas the bleeding time of the control group (the saline treated group) was about 15 minutes. On the other hand, the bleeding time of the compound A was about 15 minutes, in which it was revealed that the bleeding time of the control group was not extended or shortened. As a result, it was suggested that the PARP inhibitor did not influence to the blood coagulation system and the fibrinolytic system at all.

### 2. Effect of PARP Inhibitor on Vascular Endothelial Cell Death

A normal human umbilical cord vein endothelial cell (Cat. No. CRL-1730; Dainippon Pharmaceutical Co., Ltd.) was cultured in serum-containing liquid culture medium for vascular endothelial cell (Nissui Pharmaceutical Co., Ltd.) under conditions of 37°C, 5% CO₂. The exchange of the culture fluid was performed every other day. The cells proliferated to confluent state were sub-cultured. 5x10⁴ cells/mL of cell suspension was disseminated in an amount of 500 µL/well to a gelatin-coated 24 well plate (Iwaki). A few days later, the culture medium was removed after confirming that the cells were proliferated to confluent state, and changed to 480 µL/well of Dulbecco's Modified Eagle's Medium (DMEM; GIBCO-BRL)). The compound A was adjusted to become 50 times larger than the addition final concentration in DMEM after the dissolution of the compound A to dimethyl sulfoxide (DMSO). The sterilization was performed by using 0.22 µm filter for filtration sterilization (Millex, MILLIPORE). The 10 µL/well of DMSO solution of the compound A whose concentration was adjusted was added to cell, and 15 minutes later, 10 µL/well of 25 mM (final concentration 0.5 mM) hydrogen peroxide was added. Then, the solution was incubated under conditions of 37°C and 5% CO₂ for 6 hours.

The evaluation was performed by means of WST-1 method (modulated MTT method) with the use of Cell Counting Kit (DOJINDO). In particular, 50 µL/well of Cell Counting Reagent was added and was incubated under conditions of 37°C and 5% CO₂ for about 2 hours, after the induction of cell death by means of the aforementioned method. After that, 200 µL/well of the culture supernatant was collected, and then the absorbance (450 nm) was measured. The cell death inhibitory rate of the test substance treated group was calculated, regarding that the cell survival rate of the control group (untreated group) as a standard (100%).

As a result, the compound A showed an inhibitory effect of the vascular endothelial cell disorder dose-dependently. Thus, it was suggested that the PARP inhibitor provides the vascular endothelial cell protecting effect.

### 3. Effect of PARP Inhibitor on Cerebral Hemorrhage in Middle Cerebral Artery Occlusion-Recanalization model

### 3-1. Preparation of Middle Cerebral Artery Occlusion-Recanalization Model with Use of Male Spontaneously Hypertensive Rat

For the animal, a male spontaneously hypertensive rat (hereinafter abbreviated to SHR) (14 to 15 week old); Japan Charles River) was used. The preparation of middle cerebral artery (hereinafter abbreviated to MCA) occlusion-recanalization model of rat was performed according to the method of Koizumi, et al., (in Stroke 8: 1-7, 1986). In particular, the rat was subjected to anesthetic induction by 4% halothane (halothane vaporizer, Shinano Co., Ltd.) in 30% oxygen/70% air. The anesthesia was maintained by means of 2.5% halothane. The animal was placed in a dorsal position. The dissection of anterior neck midline was performed, the left side of the common carotid artery and the external carotid artery were exfoliated from the surrounding connective tissue around the left carotid artery bifurcation, and then a thread was hooked around the artery to ligate, respectively. Afterwards, the left internal carotid artery was exfoliated from the surrounding connective tissue, and then a thread was hooked around the artery. The range of about 5 mm from the tip of a 4-0 nylon thread for surgery (NC sterilization operation thread, Nichiyo industry Co., Ltd.) (25 mm long) was silicon-coated with an impression material for dentistry (mixture solution af XANTOPREN VL plus and OPTOSIL-XANTOPREN ACTIVATOR, Heraeus Dental Material Co., Ltd.). Then the thread was inserted to the left carotid artery from the left carotid artery bifurcation. Then, the origin part of the left MCA was occluded by means of fixing with a Sugita type clip (Mizuho Medical Industry Co., Ltd.). Nine hours after the occlusion, rat was subjected to anesthetic induction with 4% halothane in 30% oxygen /70% air. The anesthesia was maintained by means of 2.5% halothane. The plug was withdrawn, the blood flow of MCA was made restarted, and finally, the left internal carotid artery was ligated with the thread having been hooked around in advance. Neurological symptoms were observed just before recanalization, and only the individuals that indicated 5 or more points in a score of neurological symptoms were subjected to recanalization and used for the evaluation. As for a sham operation (sham), the common carotid artery, the external carotid artery and the internal carotid artery on the left side were exfoliated from the surrounding connective tissue, and then a thread was hooked around the artery to ligate, respectively. The continuous intravenous administration of saline or compound A (3 mg/kg/h) was performed for 9 hours right after the occlusion (flow rate; 10 mL/kg/h). An amount of cerebral hemorrhage was measured 24 hours after the MCA occlusion.

### 3-2: Measurement of amount of cerebral hemorrhage

An amount of cerebral hemorrhage was measured on the basis of the content of intracerebral hemoglobin (Hb) as an indicator according to a method of Asahi et al. (J Cereb Blood Flow Metab 20: 452-457, 2000)). In particular, systemic perfusion was performed with saline 24 hours after the MCA occlusion, the brain was resected, and the left (the ischemia side) cerebral hemisphere was gathered. Three mL of phosphate buffer solution was added to the left cerebral hemisphere, and the cerebral hemisphere was homogenated by means of a tissue homogenizer for 30 seconds and then an ultrasonic homogenizer for 60 seconds in sequence. Afterwards, it was centrifuged at 13,000 rpm for 30 minutes, whose supernatant was gathered as a sample for Hb measurement. Then, 160 µL of Drabkin's reagent was added to 40 µL of the sample for Hb measurement and the reaction in which Hb (mixture of oxidized form and reduced form) was converted into cyanmet Hb, was preceded at room temperature for 15 minutes. Afterwards, absorbance was measured at 540 nm of photometry wavelength. As standard substance, the cerebral hemisphere of a normal rat subjected to the operation described above after the addition of 0, 2, 4, 8, 16, 32, 64 and 128 µL of blood, was used.

As a result, the content of intracerebral hemoglobin of a sham operation group, an saline treated group, and a compound A treated group were 13.0 ± 1.1, 25.5 ± 2.2, 17.7 ±1.3 µL, respectively, and the compound A treated group exhibited a significant inhibitory effect for increase of the amount of cerebral hemorrhage in a cerebral hemorrhage model with the use of SHR. Thus, it was suggested that a PARP inhibitor reduced hemorrhage in cerebrovascular disorder.

In addition, intracerebral hemoglobin content can be also measured by using the following methods, in particular, an enzyme-linked immunosorbent assay (ELISA). Systemic perfusion was performed with saline 24 hours after the MCA occlusion, the brain was resected, and the left cerebral hemisphere (the ischemia side) was gathered. Three mL of phosphate buffer solution (hereinafter abbreviated to PBS) was added to the left cerebral hemisphere, and the cerebral hemisphere was homogenated by means of a tissue homogenizer for 30 seconds and then an ultrasonic homogenizer for 60 seconds in sequence. Afterwards, it was centrifuged at 13,000 rpm for 30 minutes, whose supernatant was gathered as a sample for Hb measurement. A sample (which was diluted by 100 times with PBS containing 0.05% Tween 20 and 1% bovine serum albumin (BSA)) (hereinafter abbreviated to1% BSA-PBST), and hemoglobin (5, 10, 20, 40, 80 and 160 ng/mL) prepared as a standard solution, in an amount of 100 µL/well, were added to a 96 well plate, then left overnight at 4°C. It was washed with 0.05% Tween 20-containing PBS three times, added 1% BSA-PBST in an amount of 200 µL/well, and left at room temperature for 30 minutes. Anti-hemoglobin rabbit polyclonal antibody (Biogenesis, diluted by 5,000 times in, 1% BSA-PBST) was added in an amount of 100 µL/well, and left at room temperature for an hour.

It was washed with 0.05% Tween 20-containing PBS 3 times, a horseradish peroxidase (HRP) labeled goat anti-rabbit IgG antibody (Bio-Rad, diluted by 2,500 times in, 1% BSA-PBST) was added in an amount of 100 µL/well and left at room temperature for an hour. Then it was washed 4 times with 0.05% Tween 20-containing PBS, then peroxidase substance solution (R & D Systems) was added in an amount of 100 µL/well. It was left at room temperature for 5 minutes, added 200 mmol/L hydrochloric acid in an amount of 100 µL/well as stop solution, and then the absorbance (450 nm) was measured. Microplate Reader SPECTRA MAX 250 (Japan Molecular Devices Co., Ltd.) was applied to the measurement of absorbance.

As a result, the content of intracerebral hemoglobin of a sham operation group, an saline treated group, and a compound A treated group (1,3,10 mg/kg/h) were 4.5±0.74, 19.6±2.21, 15.4±2.19, 15.5±2.14, 10.3±1.24 µg/mL, respectively, and the compound A treated group exhibited a significant inhibitory effect on the increase of the amount of cerebral hemorrhage in a cerebral hemorrhage model with the use of SHR. Thus, it was suggested that a PARP inhibitor reduced hemorrhage in cerebrovascular disorder.

An amount of intracerebral hemoglobin can be measured by means of the above-mentioned methods, but was not limited to these.

### 4. Effect of PARP Inhibitor on Blood-Brain Barrier Failure in Hemorrhagic Cerebral Infarction Model with the use of SHR

According to "Preparation of Cerebral Artery Occlusion-Recanalization Model with Use of Male Spontaneously Hypertensive Rat" in the above 3-1, a hemorrhagic cerebral infarction model with the use of SHR is prepared.

Permeability of a blood-brain barrier is measured with the use of Evans blue extraction method. In particular, as a tracer of permeability of the blood-brain barrier, 2% Evans blue in a volume of 4 mL/kg was administrated intravenously. One hour after the Evans blue administration, systemic perfusion was performed with saline and the brain is removed. The removed brain are divided into right and left cerebral hemispheres, 3 mL of 50% trichloroacetic acid is added, and then the hemispheres are homogenated with a tissue homogenizer. The homogenated product is centrifuged at 4°C, 12,000 rpm for 20 minutes and then an absorbance (620 nm) of the supernatant is measured. Microplate Reader SPECTRA MAX 250 (Japan Molecular Devices Co., Ltd.) is applied for the measurement of absorbance.

After the MCA occlusion, an amount of Evans blue leakage in a brain in 3, 6,9, 12, 18 and 24 hours were measured and an effect of a PARP inhibitor was evaluated.

### 5. Effect of PARP Inhibitor on Intracerebral Hemorrhage Model by t-PA Administration

The preparation of PIT (Photochemically induced thrombosis) model is performed according to the method of Umemura et al. In particular, anesthesia is maintained to a rat by 2.5% halothane inhalation in 30% oxygen /70% air. Skin is incised along the left side of orbit, the temporal muscle is incised in part, and the infratemporal fossa is incised along the lateral wall of the orbit. The skull base is incised in an oval shape window of about 3mm with a dental drill under an operating microscope. MCA is observed through a dura matter in a window. Green light irradiation Probe (Hamamatsu Photonics K.K.) with an absorption maximum wavelength on MCA to 540 nm is set on MCA. Rose Bengal (Wako Pure Chemical Industries, Ltd.) (10-20 mg/kg) is administered from a tail vein, and green light (40,000 luxes) is irradiated right after the administration to induce thrombus to MCA. Complete occlusion of MCA is confirmed under an operating microscope, and then the surgical wound is closed.

The hemoglobin content is measured by the same method as the above described 3-2, and the evaluation of the effectiveness of the PARP inhibitor is performed under the conditions that the intracerebral hemorrhage by t-PA is confirmed on the basis of the intracerebral hemoglobin content.

### Formulation Examples

The typical formulation examples used for the present invention are shown below.

### Formulation Example 1

Compound A (100g), calcium carboxymethyl cellulose (disintegrant, 10.0 g), magnesium stearate (lubricants, 10.0 g) and microcrystalline cellulose (870 g) were mixed by a conventional method and then compressed to obtain 10,000 tablets each containing 10 mg of an active ingredient.

### Formulation Example 2

Compound A (100 g), mannitol (2 kg) and distilled water (50 L) were mixed by a conventional method and filtered with a dust filter, and then each ampoule was filled with 5 mL of the obtained mixture and subjected to heat sterilization in an autoclave to obtain 10,000 ampoules each containing 10 mg of an active ingredient.

### INDUSTRIAL APPLICABILITY

The PARP inhibitor may reduce hemorrhage in cerebrovascular disorder by means of the inhibitory effect of the vascular endothelial cell disorder without affecting the blood coagulation system and the fibrinolytic system. In addition, the PARP inhibitor with the combination of a thrombolytic agent inhibits the hemorrhage that is concerned about at the point of use of a thrombolytic agent, and TTW extension effect of a thrombolytic agent can be also expected.

## Claims

1. A hemorrhage reducing agent in cerebrovascular disorder, comprising a poly(ADP-ribose) polymerase inhibitor.

2. The agent according to claim 1, wherein the poly(ADP-ribose) polymerase inhibitor is one or more compound(s) selected from GPI-15427; GPI-16539; GPI-18078; GPI-6000; GPI-6150; KU-0687; INO-1001; FK-866; 4-(4-(N,N-dimethylaminomethyl)phenyl)-5-hydroxyisoquinolinone, FR-255595; FR-257516; FR-261529; FR-247304; M-50916; ABT-472; ONO-1924H; DR-2313; CEP-8983; AG-014699; BGP-15; AAI-028; PD-141076; PD-141703; a compound described in specifications of WO03/070707, WO00/044726, WO00/42,040, WO01/16136, WO01/42219 and WO01/70674.

3. The agent according to claim 1, wherein the poly(ADP-ribose) polymerase inhibitor is a compound represented by formula (I): 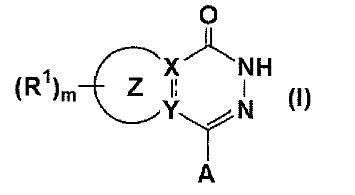 wherein R¹ represents (1) hydrogen atom, (2) C1-8 alkyl, (3) C1-8 alkoxy, (4) hydroxyl, (5) halogen atom, (6) nitro, (7) NR²R³, (8) C2-8 acyl, (9) C1-8 alkoxy substituted with phenyl, or (10) C2-8 acyl substituted with NR²R³; R² and R³ each independently represents (1) hydrogen atom or (2) C1-8 alkyl; X and Y are each independently represents (1) C, (2) CH or (3) N;
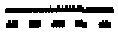 represents (1) a single bond or (2) a double bond; 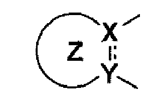 represents (1) C3-10 monocyclic carbocyclic aryl, which may be partially or fully saturated, or (2) 3- to 10-membered monocyclic heterocyclic aryl, which may be partially or fully saturated, containing 1 to 4 heteroatom(s) which is selected from oxygen atom, nitrogen atom and sulfur atom; and A represents (1) A¹, (2) A², (3) A³, (4) A⁴ or (5) A⁵;
A¹ represents 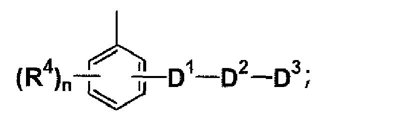 A² represents -E¹ -E² -E³-E⁴;
A³ represents 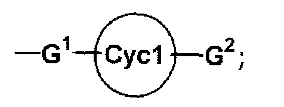 A⁴ represents 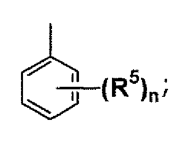 A⁵ represents 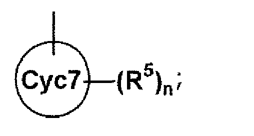 D¹ represents (1) -NR⁶C(O)-, (2) -NR⁶C(S)-, (3) -NR⁶SO₂-, (4) -CH₂-NR⁶-group, (5) -CH₂-O-, (6) -OC(O)-, (7) -CH₂-NR⁶C(O)-, (8) -NR⁶C(O)NR⁷-, (9) -NR⁶C(O)O-, (10) - NR⁶C(S)NR⁷-, (11) -NR⁶-, or (12) -NR⁶C(=NR⁷)-; R⁶ and R⁷ each independently represents (1) hydrogen atom, (2) C1-8 alkyl, (3) phenyl or (4) C1-8 alkyl substituted with phenyl; D² represents (1) C1-8 alkylene, (2) C2-8 alkenylene, (3) Cyc2, (4) -(C1-4 alkylene)-O-(C1-4 alkylene)-, (5) -(C1-4 alkylene)-S-(C1-4 alkylene)-, (6) -(C1-4 alkylene)-NR⁸-(C1-4 alkylene)-, (7) -(Cyc2)-(C1-8 alkylene)-, (8) -(C1-8 alkylene)- (Cyc2)-, or (9) -(C1-4 alkylene)-(Cyc2)-(C1-4 alkylene)-; R⁸ represents (1) hydrogen atom, (2) C1-8alkyl, (3) C1-8 alkoxycarbonyl, (4) phenyl or (5) C1-8 alkyl substituted with phenyl; D³ represents (1) hydrogen atom, (2) -NR⁹R¹⁰, (3) Cyc3, (4) -OR¹¹, (5) COOR¹², (6) CONR¹³R¹⁴, (7) cyano, (8) halogen atom, (9) -C(= CR¹⁵)NR¹⁶R¹⁷, or (10) -NR¹⁸C(= NR¹⁹)NR²⁰R²¹; R⁹ and R¹³ each independently represents (1) hydrogen atom, (2) C1-8 alkyl, (3) C2-8 alkenyl, (4) C2-8 alkynyl, (5) Cyc3, (6) C1-8 alkoxy, (7) C2-8 alkenyloxy, (8) C2-8 alkinyloxy, or (9) C1-8 alkyl substituted with Cyc3, C1-8 alkoxy, C1-8 alkylthio, cyano, hydroxyl or 1 to 3 halogen atom(s); R¹⁰ and R¹⁴ each independently represents (1) hydrogen atom, (2) C1-8 alkyl, (3) C2-8 alkenyl, (4) C2-8 alkynyl, (5) C1-8 alkoxycarbonyl, (6) C2-8 acyl, (7) C3-8 cycloalkyl, (8) C1-8 alkoxycarbonyl substituted with Cyc4 or 1 to 3 halogen atom(s), or (9) C1-8 alkyl substituted with C1-8 alkoxy; R¹¹ and R¹² each independently represents (1) hydrogen atom or (2) C1-8 alkyl; R¹⁵, R¹⁶, R¹⁷, R¹⁸, R¹⁹, R²⁰ and R²¹ each independently represents (1) hydrogen atom, (2) C1-8 alkyl, (3) C1-8 alkoxycarbonyl, (4) phenyl or (5) C1-8 alkyl substituted with phenyl; R⁴ represents (1) hydrogen atom, (2) C1-8 alkyl, (3) C1-8 alkoxy, (4) hydroxyl, (5) halogen atom, (6) nitro or (7) NR²²R²³; R²² and R²³ each independently represents (1) hydrogen atom or (2) C1-8 alkyl; E¹ represents C1-4 alkylene, E² represents (1) -C(O) NR²⁴-, (2) -NR²⁴C (O)-, (3) -NR²⁴-, (4) -C(O)O- or (5) -S-; R²⁴ represents (1) hydrogen atom, (2) C1-8 alkyl or (3) C1-8 alkyl substituted with phenyl; E³ represents (1) a single bond or (2) C1-8 alkylene; E⁴ represents (1) C1-8 alkyl, (2) C2-8 alkenyl, (3) C2-8 alkynyl, (4) Cyc5, (5) NR²⁵R²⁶, (6) OR²⁷, (7) SR²⁷, (8) COOR²⁷, (9) C1-8 alkyl substituted with two OR²⁵s, (10) C1-8 alkyl substituted with 1-3 halogen atoms, (11) cyano, or (12) C2-8 acyl; R²⁵ represents (1) hydrogen atom, (2) C1-8 alkyl, (3) C2-8 alkenyl, (4) C2-8 alkynyl, (5) Cyc5 or (6) C1-8 alkyl substituted with Cyc5 or OR²⁸; R²⁶ represents (1) hydrogen atom, (2) C1-8 alkyl, (3) C1-8 alkoxycarbonyl, (4) phenyl or (5) C1-8 alkyl substituted with phenyl; R²⁷ represents (1) hydrogen atom, (2) C1-8 alkyl, (3) Cyc5 or (4) C1-8 alkyl substituted with Cyc5; R²⁸ represents (1) hydrogen atom or (2) C1-8 alkyl; G¹ represents C1-8 alkylenes; Cyc1 represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom; G² represents (1) hydrogen atom, (2) C1-8 alkyl, (3) C1-8 alkoxycarbonyl, (4) C2-8 acyl, (5) Cyc6, (6) C1-8 alkyl or C2-8 alkenyl substituted with 1 to 2 of Cyc6, hydroxyl or the C1-8 alkoxy, (7) C1-8 alkoxycarbonyl substituted with Cyc6, (8) -C(O)-Cyc6, (9) nitro, (10) NR⁴¹R⁴², (11) C1-8 alkoxy, or (12) C1-8 alkyl substituted with NR⁴¹R⁴²; R⁴¹ and R⁴² each independently represents (1) hydrogen atom or (2) C1-8 alkyl; R⁵ (1) hydrogen atom, (2) C1-8 alkyl, (3) C1-8 alkoxy, (4) hydroxyl, (5) nitro, (6) NR²⁹R³⁰, (7) C1-8 alkyl substituted with NR²⁹R³⁰, (8) NHSO₂OH, (9) amidino, (10) cyano, (11) halogen atom, (12) Cyc8, or (13) C1-8 alkyl substituted with Cyc8; R²⁹ and R³⁰ each independently represents (1) hydrogen atom or (2) C1-8 alkyl; Cyc2, Cyc3, Cyc4, Cyc5, Cyc6 and Cyc8 each independently represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom; Cyc7 represents (1) C3-10 monocyclic or bicyclic carbocyclic aryl, which may be partially or fully saturated, or (2) 3- to 10-membered monocyclic or bicyclic heterocyclic aryl, which may be partially or fully saturated, containing 1 to 4 heteroatom(s) which is selected from oxygen atom, nitrogen atom and sulfur atom, provided that Cyc7 does not represent benzene ring, Cyc2, Cyc3, Cyc4, Cyc5, Cyc6 and Cyc8 may be substituted with 1 to 3 group(s), which is selected from (1) C1-8 alkyl, (2) C2-8 alkenyl, (3) C1-8 alkoxy, (4) halogen atom, (5) trihalomethyl, (6) trihalomethoxy, (7) C1-8 alkoxycarbonyl, (8) oxo, (9) C1-8 alkyl substituted with the C1-8 alkoxy or phenyl, (10) hydroxyl, or (11) NR²⁹R³⁰; and m and n each independently represents 1 or 2, or a salt, an N-oxide or solvate thereof, or a prodrug thereof.

4. The agent according to claim 3, wherein the compound represented by formula (I) is 4-(N-(4-(morpholin-4-yl)butyl)carbamoylmethyl)-5,6,7,8-tetrahydrophthalazin-1(2H)-one.

5. The agent according to claim 1, wherein the hemorrhage is caused by antithrombotic treatment.

6. The agent according to claim 1, wherein the hemorrhage is caused by one or more agent(s) selected from a thrombolytic agent, an anticoagulant agent and an antiplatelet agent.

7. The agent according to claim 1, which is an agent for inhibiting a vascular endothelial cell disorder.

8. The agent according to claim 7, which is an agent for repair and/or disrupting inhibition of blood-brain barrier,

9. The agent according to claim 1, which is an agent for extending therapeutic time window of a thrombolytic agent.

10. The agent according to claim 1, which is an agent for prevention and/or treatment of cerebrovascular disorder.

11. The agent according to claim 1, which is an agent for prevention and/or treatment of hemorrhagic complication due to antithrombotic treatment.

12. A hemorrhage reducing agent in cerebrovascular disorder, comprising a poly(ADP-ribose) polymerase inhibitor used in combination with one or more agent(s) selected from a thrombolytic agent, an anticoagulant agent, an antiplatelet agent, a cerebroprotection agent, an anticerebral edema agent, a plasma expander, an immunosuppressive agent, an intercellular an adhesion factor inhibitor, an interleukin-8 antagonist and a steroid.

13. The agent according to claim 12, wherein the agent is a concomitant agent.

14. The agent according to claim 13, wherein the poly(ADP-ribose) polymerase inhibitor is used in combination with one or more agent(s) selected from a thrombolytic agent, an anticoagulant agent and an antiplatelet agent.

15. The agent according to claim 14, wherein the poly(ADP-ribose) polymerase inhibitor is 4-(N-(4-(morpholin-4-yl)butyl)carbamoylmethyl)-5,6,7,8-tetrahydrophthalazin-1(2H)-one, and the thrombolytic agent is t-PAs.

16. The agent according to claim 1, wherein the hemorrhage is bleeding in a hemorrhagic cerebrovascular disease and/or bleeding involved in ischemic-reperfusion.

17. The agent according to claim 1, which is an agent for protecting a vascular endothelial cell.

18. A method for reducing hemorrhage in cerebrovascular disorder in mammals, comprising administering effective dose of a poly(ADP-ribose) polymerase inhibitor to the mammals.

19. A use of a poly(ADP-ribose) polymerase inhibitor for producing a hemorrhage reducing agent in cerebrovascular disorder.

20. A method for reducing hemorrhage in cerebrovascular disorder in mammals, comprising administering effective dose of 4-(N-(4-(morpholin-4-yl)butyl)carbamoylmethyl)-5,6,7,8-tetrahydrophthalazin-1(2H)-one used in combination with effective dose of t-PAs to mammals.

21. A use of 4-(N-(4-(morpholin-4-yl)butyl)carbamoylmethyl)-5,6,7,8-tetrahydrophthalazin-1(2H)-one and t-PAs for producing a hemorrhage reducing agent in cerebrovascular in which one or more agent(s) are used in combination.
